# EUROPEAN PATENT APPLICATION

(11) **EP 2 745 833 A1**
(43) Date of publication of application: **25.06.2014**
(21) Application number: 12306648.2
(22) Date of filing: 21.12.2012
(51) Int. Cl.: A61K 9/00, A61K 9/20

(54) **Soluble, dispersible or orodispersible tablets comprising cyclophosphamide**

(71) Applicant: Institut Gustave Roussy, 94805 Villejuif Cédex (FR); Negositek, 33300 Bordeaux (FR); Andrieu Consultant International, 13008 Marseille (FR)
(72) Inventor: Vassal, Gilles, 75011 Paris (FR); Saslawski, Olivier, 33300 Bordeaux (FR); Andrieu, Véronique, 13008 Marseille (FR)
(74) Representative: Casalonga

(57) **Abstract**

The present invention concerns a soluble tablet, which comprises:
(a) cyclophosphamide, a hydrate form thereof, a pharmaceutically acceptable salt thereof, or any mixture thereof, in an amount ranging from 1 to 15 % by weight on a cyclophosphamide active weight basis, and
(b) from 10% to 99 % by weight of a solubilising agent chosen from disaccharides, sugar alcohols that are solid at ambient temperature, and mixtures thereof.

The tablets of the invention can be dispersed in water, and allow to administer cyclophosphamide in a safe and reliable manner. They are particularly suitable for treating paedriatric cancer. They can also be dissolved in saliva.

## Description

The present invention relates to a novel pharmaceutical form for administering cyclophosphamide to a patient, and in particular to a soluble, dispersible or orodispersible tablet that allows to deliver cyclophosphamide in a very convenient, efficient and safe manner. Such a form is particularly interesting for paediatric applications.

Cyclophosphamide is a well known drug that has been used for many years in cancer treatment. Also known as cytophosphane, it is an alkylating agent from the oxazophorine group which is used to treat various types of cancer as well as some autoimmune disorders. As a "prodrug", it is converted in the liver to active metabolites that have chemotherapeutic activity.

At present, there are two main dosage forms available on the market, which are coated tablets (containing generally 50 mg of cyclophosphamide) and parenteral formulations (so called "IV formulations"), in which the cyclophosphamide is under the form of a powder (500 mg or 1000 mg) comprising sodium chloride that has to be dissolved in a solution for injection before administration by perfusion (from 30 min to 2 hours or over 24 hours).

If current formulations can be suitable for adults, they are unfortunately not adapted for pediatrics. Indeed, children usually depending on their age, cannot or do not like to swallow tablets, and intravenous administration is quite traumatic for young children. This situation is particularly critical for new born babies aged of some months. In addition, chemotherapy intravenous administration is not appropriate for ambulatory treatments, whereas children have to take a daily dose over months.

Therefore, cyclophosphamide is generally administered to children using the powder for IV formulations, by dissolving the required amount of powder in water, and having the aqueous solution drunk by the patient.

However, the dissolution in water of the cyclophosphamide powder is slow, and the solution obtained is under the form of a cloudy suspension rather than a real solution. Even after a proper stirring, once the solution has been drunk some of the product often remains in the recipient, so that it is difficult to ensure that the exact proper amount has been administered to the patient. In addition, in order to be absorbed by the gastrointestinal tract of the patient, any active ingredient needs to be as fully as possible dissolved under molecular state.

Furthermore, once dissolved in solution the IV formulation should be immediately administered and must not be kept more than 48 hours as cyclophosphamide is not stable on a long term in aqueous solutions.

Therefore, oral administration of cyclophosphamide using dissolved powder is always performed under strict control at the hospital; all the more that cyclophosphamide is a cytotoxic compound that cannot be manipulated without specific care. This requires that the child comes to the hospital every day over months.

Thus, there is an urgent need to provide a new dosage form of cyclophosphamide, which could facilitate the administration thereof in particular in the case of children, while being safe and efficient.

The Applicant has now discovered that cyclophosphamide when mixed with particular solubilizing agents could be administered either under the form of soluble or dispersible tablets that have the unexpected property of allowing a very quick dissolution of cyclophosphamide in a very small volume of water, or under the form of orodispersible tablets that have the unexpected property of allowing a very quick dissolution of cyclophosphamide in a very small volume of saliva in the mouth.

Hence, in presence of said solubilizing agents, the cyclophosphamide can be very efficiently dissolved in a very short time, much shorter than cyclophosphamide drug substance alone, and in a small amount of water or of aqueous medium such as saliva.

Indeed, as mentioned above the dissolution of cyclophosphamide powder in water is long (at least 15 to 20 minutes, and sometimes up to 30 minutes), whereas the dispersion or dissolution of the tablets of the invention takes less than 2 minutes, and depending on the formulation can take less than 1 minute.

The present invention therefore concerns a soluble, dispersible or orodispersible tablet, which comprises:
(a) cyclophosphamide, a hydrate form thereof, a pharmaceutically acceptable salt thereof, or any mixture thereof, in an amount ranging from 1 to 15 % by weight on a cyclophosphamide active weight basis, and
(b) from 10 to 99 % by weight of a solubilising agent chosen from disaccharides, sugar alcohols that are solid at ambient temperature, and mixtures thereof.

Such a tablet allows to prepare a solution of cyclophosphamide in water in a short time and in a small amount of water, which is very convenient for treating patients that cannot swallow neither coated tablets nor large amounts of water, such as in particular babies and young children, as well as old persons. The tablets of the invention further facilitate the administration of cyclophosphamide to any kind of patient.

The tablets of the invention can be dispersed or dissolved rapidly and completely in water at ambient temperature, leading to a solution of cyclophosphamide that is clear to opaque, but that does not contain remaining non dissolved particles of cyclophosphamide, so that the total amount of cyclophosphamide present in the tablet is effectively absorbed as a solution. Therefore the tablets of the invention constitute a very reliable and efficient way of administering cyclophosphamide.

They are particularly suitable for treatments that are carried out at home, and allow a dosing of cyclophosphamide that is very convenient while being safe for the environment as well as for the patients and eventual caregivers such as parents, nurses...

Such tablets enhance patient compliance and improve access to safe and efficient anticancer medicines in paediatric oncology, particularly in infants and very young children.

The tablets of the invention can also be dispersed rapidly and completely in saliva, and can therefore be under the form of orodispersible tablets, which facilitates the ease and comfort of administration of the drug, while ensuring that the proper amount is effectively absorbed by the patient.

The present invention further provides a process for manufacturing tablets as disclosed herein.

The present invention further concerns the use of the solubilising agents disclosed therein, for improving the dissolution of cyclophosphamide in aqueous solutions or beverages, such as water and saliva.

The present invention also concerns a soluble, a dispersible and an orodispersible tablet as herein disclosed, for its use in treating cancer or autoimmune disorders.

Such tablets can be used to treat a wide range of neoplastic conditions, including leukaemias, lymphomas, soft tissue and osteogenic sarcomas, malignancies and solid tumours. They can be used in combination chemotherapy regimens with other cytotoxic drugs.

The tablets of the invention can of course be administered to every patient that needs to be treated with cyclophosphamide. Use thereof is particularly suitable for patients that have difficulties to swallow such as children.

They can be used in long term treatments, in particular according to a daily administration.

The tablets according to the invention are soluble, dispersible or orodispersible.

By "soluble" it is meant that the tablet of the invention can be completely dissolved in water at ambient temperature, leading to a clear solution in which all ingredients of the tablet, including the cyclophosphamide, are fully dissolved.

By "dispersible", it is meant that the tablet of the invention can be dispersed in water at ambient temperature, leading to an opaque solution or suspension in which the cyclophosphamide is fully dissolved. The unclear aspect of the suspension is due to the incomplete dissolution of one or more of the excipient(s) present in the tablet.

By "orodispersible", it is meant that the tablet of the invention can be dissolved or dispersed in saliva at body temperature, leading to a solution or suspension in which the cyclophosphamide is fully dissolved in saliva.

By "cyclophosphamide", it is meant according to the present invention the 2-[bis(2-chloroethyl)amino]tetrahydro-2H-1,3,2-oxazaphosphorine 2-oxide, corresponding to the following formula (I):

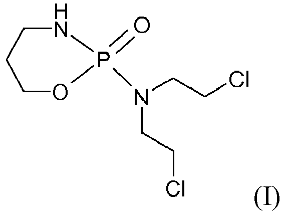

In the tablet of the invention, cyclophosphamide can be used under its anhydrous form (corresponding to formula (I) above), as well as under any of its hydrate forms, any of its pharmaceutically acceptable salts, and any mixture of such forms.

According to a preferred embodiment, the cyclophosphamide is employed under the form of the monohydrate compound.

The tablets of the invention comprise an amount of cyclophosphamide, hydrate form(s) thereof, pharmaceutically acceptable salt(s) thereof, or a mixture thereof, which ranges from 1 to 15 % by weight, preferably from 2 to 10% by weight, more preferably from 4 to 8 % by weight, on a cyclophosphamide active weight basis.

The particularly preferred amount is 6.25% by weight, on a cyclophosphamide active weight basis.

In the present disclosure, except specifically indicated otherwise, all amounts are expressed in amounts by weight, or in percentages by weight relative to the total weight of the tablet.

With regard to cyclophosphamide, the amounts are expressed by weight, on an anhydrous cyclophosphamide weight basis. For example, when the cyclophosphamide used in the tablet is under the form of a salt, "5 % by weight of the cyclophosphamide on an anhydrous cyclophosphamide weight basis" means that the amount of the cyclophosphamide salt used in the tablet is that which corresponds to 5% by weight of cyclophosphamide in anhydrous form (formula (I) above).

The cyclophosphamide is advantageously dispersed or diluted, preferably in a homogeneous manner, in the soluble tablets of the invention.

The tablets of the present invention contain at least one solubilising agent, which is chosen from disaccharides, sugar alcohols that are solid at ambient temperature, and mixtures thereof.

Among the disaccharides that are preferably used, mention can be made in particular of lactose.

The sugar alcohols used in the tablet of the invention are those which are solid at ambient temperature (25°C).

They are preferably chosen from sugar alcohols containing at least 4 carbon atoms, more preferably at least 5 carbon atoms.

Among the sugar alcohols that are preferably used, mention can be made in particular of mannitol, sorbitol, xylitol, isomalt, maltitol, and mixtures thereof.

According to a preferred embodiment, the solubilising agent contains at least one sugar alcohol that is solid at ambient temperature, and more preferably the solubilising agent contains mannitol.

According to a particularly preferred embodiment, the solubilising agent consists in one or more sugar alcohol(s) that is (are) solid at ambient temperature. Even more preferably, the solubilising agent is mannitol.

The tablets of the invention contain from 10% to 99 % by weight of said solubilising agent, based on the total weight of the tablet.

More preferably, the amount of solubilising agent in the tablet ranges from 10 to 98% by weight, more preferably from 65 to 95 % by weight, even more preferably from 70 to 90 % by weight.

The tablets of the invention can further contain any pharmaceutical excipient, provided it is compatible with cyclophosphamide. In particular, such excipients should not lead to degradation of cyclophosphamide either in the tablet or in water, and should not slow the dissolution thereof or reduce in any way the dissolving activity of the solubilizing agent.

In particular, the tablets of the invention can further contain one or more lubricants, which can be chosen among the lubricants classically used in the manufacture of solid tablets.

Among the suitable lubricants, mention can be made for example of stearic acid as well as magnesium and calcium salts thereof, hydrogenated vegetable oil, mineral oil, polyethyleneglycol, sodium lauryl sulfate, glyceryl palmitostearate, glyceryl behenate, sodium benzoate, sodium stearyl fumarate, talc, silicon dioxide, and mixtures thereof.

A particularly preferred lubricant is magnesium stearate.

The lubricant(s) can be present in the tablets of the invention in an amount advantageously ranging from 0.1 to 5% by weight, more preferably from 0.5 to 3% by weight.

The tablets of the invention can additionally contain one or more disintegrants, which can be chosen among the disintegrants classically used in the manufacture of solid tablets.

Among the suitable disintegrants, mention can be made for example of celluloses and modified celluloses such as in particular cross-linked sodium carboxymethyl cellulose, starches and modified starches such as pregelatinized starch and carboxymethyl starch (in particular sodium starch glycolate), cross-linked povidones, and mixtures thereof.

Particularly preferred disintegrants are chosen from cross-linked carboxymethyl celluloses (such as croscarmellose sodium) and carboxymethyl starches (such as sodium starch glycolate).

According to a particularly preferred embodiment, the disintegrant is chosen from carboxymethyl starches. The Applicant has indeed discovered that tablets containing such a disintegrant exhibit particularly improved properties in terms of cyclophosphamide stability.

According to a particular preferred embodiment, the soluble tablets of the invention do not contain microcrystalline cellulose. Indeed, the Applicant has discovered that microcrystalline cellulose tends to diminish the stability of cyclophosphamide. Therefore, while being clearly not excluded from the scope of the invention, it is however preferred that microcrystalline cellulose is not employed in the tablets of the invention.

The disintegrant(s) can be present in the tablets of the invention in an amount advantageously ranging from 1 to 20% by weight, more preferably from 3 to 15 % by weight.

Among the other additional excipients, mention can be made for example of binders, fillers, diluents, sweeteners, flavouring agents, colouring agents and any other pharmaceutical excipient known in the art.

The dosage of cyclophosphamide administered to each patient generally depends on its body surface area, which depends on the weight and the height of the patient.

The administration dose for each patient depends of course on the clinical scheme, which is individual. It typically ranges from 25 to 200 mg / m² body surface per day. Examples of preferred daily doses for children are 25 mg / m² body surface, 40 mg / m² and 50 mg / m² body surface. For adolescents and adults, the preferred daily doses are 50 mg / m² body surface, 100 mg / m² body surface and 200 mg / m² body surface.

The tablets can be administered according to a continuous schedule, or according to a discontinuous schedule for example with short treatment cycles of 1 to 14 days, repeated each 2 to 4 weeks.

The tablets of the invention can advantageously be manufactured in different dosage strengths, to allow administration of cyclophosphamide to patients having very different ages and body surface areas, from new born babies to adults.

The tablets of the invention can advantageously be manufactured in dosage strengths ranging from 5 to 100 mg of cyclophosphamide, on a cyclophosphamide active weight basis. That is to say, the tablets of the invention can advantageously contain from 5 to 100 mg of cyclophosphamide, on a cyclophosphamide active weight basis.

Examples of preferred dosage strengths are tablets containing 5 mg, 10 mg, 20 mg, 40 mg, 50 mg and 100 mg of cyclophosphamide, on a cyclophosphamide active weight basis.

In particular, the tablets corresponding to the dosage strengths above allow treating a wide range of patients, all the more that they can be combined in case intermediate or additional strengths are needed.

For example, 10 mg dosage strength can be easily administered by dissolving two 5 mg tablets in a glass of water. Likewise, 70 mg dosage strength can be easily administered by dissolving a 50 mg tablet and a 20 mg tablet.

Therefore, combinations of tablets of the invention allow to manage intermediate body surface areas.

The tablets of the invention can further advantageously be divisible, for example in 2, 3 or 4 parts, in order to achieve further intermediate dosage strengths.

The table hereunder details a particularly preferred example of formulation of tablets of the invention having dosage strengths of 5 mg, 20 mg and 50 mg of cyclophosphamide.

**Table 1: Composition of the 5 mg, 20 mg and 50 mg soluble and orodispersible tablets**

| Ingredients | Centesimal composition w/w | Unit Composition (mg) | | | Function |
|---|---|---|---|---|---|
| Cyclophosphamide | 6.25% | 5.00 | 20.00 | 50.00 | Active ingredient |
| Mannitol | 86% | 68.8 | 275.2 | 688 | Diluent |
| Croscarmellose sodium | 6.25% | 5.00 | 20 | 50.00 | Disintegrant |
| Magnesium stearate | 1.50% | 1.20 | 4.80 | 12.00 | Lubricant |
| Total | 100% | 80.00 | 320.00 | 800.00 | |

The centesimal composition of the formulation is advantageously homothetic for the different dosage strengths.

The administration of the tablets of the invention is generally done by dissolving or dispersing the tablet in a glass of water (or any suitable recipient such as a feeding-bottle), just before oral administration to the patient, or in the mouth of the patient in its saliva.

The tablet can be advantageously dissolved or dispersed in the minimum volume of water required for insuring complete dissolution or dispersion thereof. This minimum volume depends on the dosage strength of the tablet, and on its composition.

In order to insure optimum dispersion of the tablet and dissolution of cyclophosphamide in water, the two following conditions should advantageously be respected:
- the minimum volume of water should be preferably at least 5 ml (this volume guaranteeing an optimally improved dispersion of the tablet and dissolution of cyclophosphamide), and
- the maximum concentration of cyclophosphamide in the aqueous solution should be 4.0 mg / ml (in order to avoid any risk of partial dissolution of the active substance before administration).

The minimum volume of water can therefore be determined as follows;

Minimum water volume = dosage strength of the tablet (mg) /4.0 (mg/ml), with a preferred minimum value of 5 ml.

Of course, a higher volume of water can be used.

The tables hereunder indicate the particular tablet units (on the basis of the three formulations detailed in Table 1 above) that can be administered to children having different body surface areas, and for different dose treatments (25, 40 and 50 mg/m² respectively). The tables hereunder further detail the minimum volume of water to be used to disperse the corresponding tablets.

### Daily dose of 25 mg/m²:

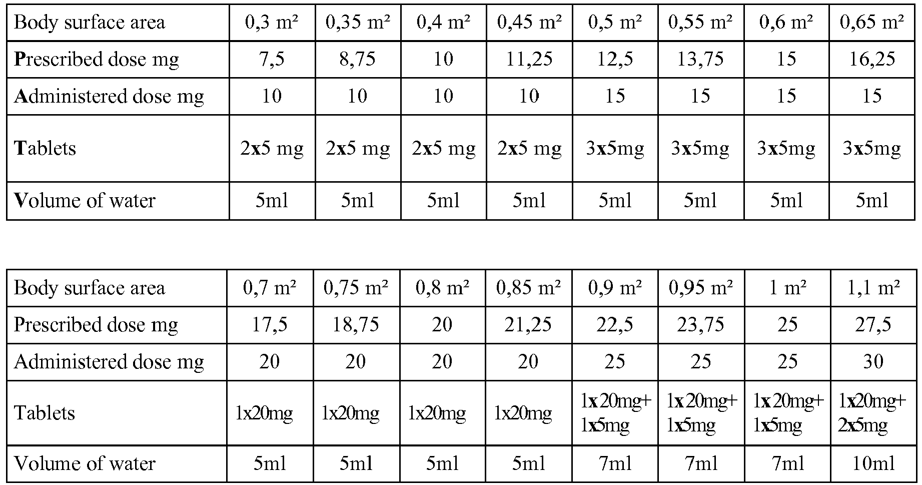

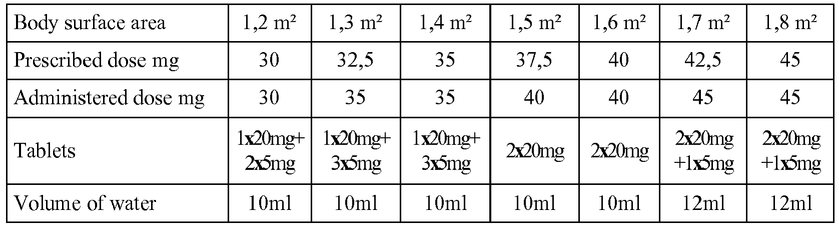

### Daily dose of 40 mg/m²:

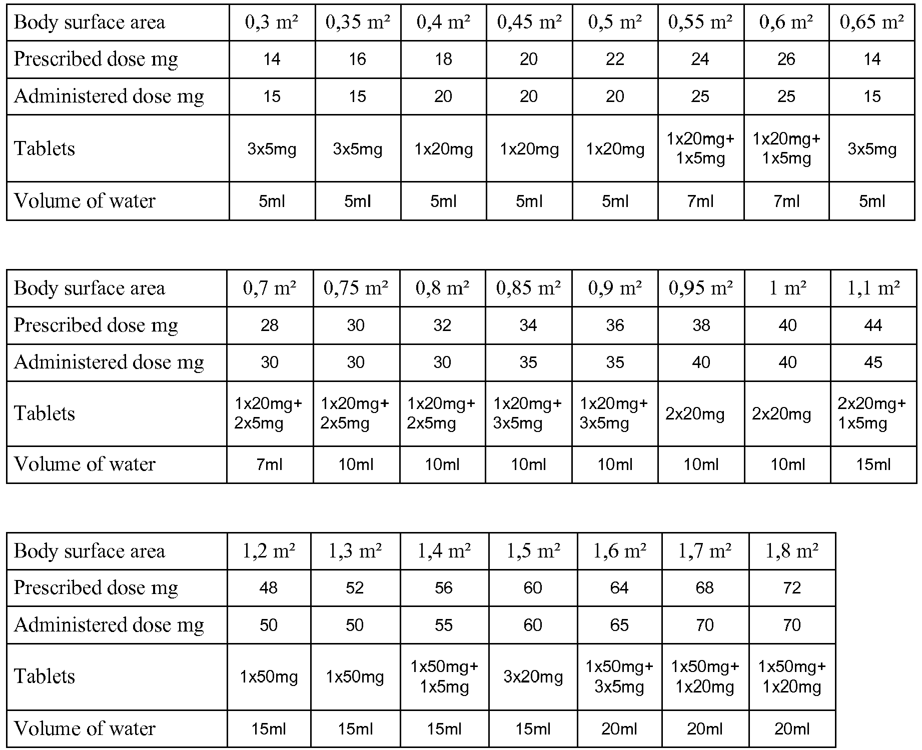

### Daily dose of 50 mg/m²:

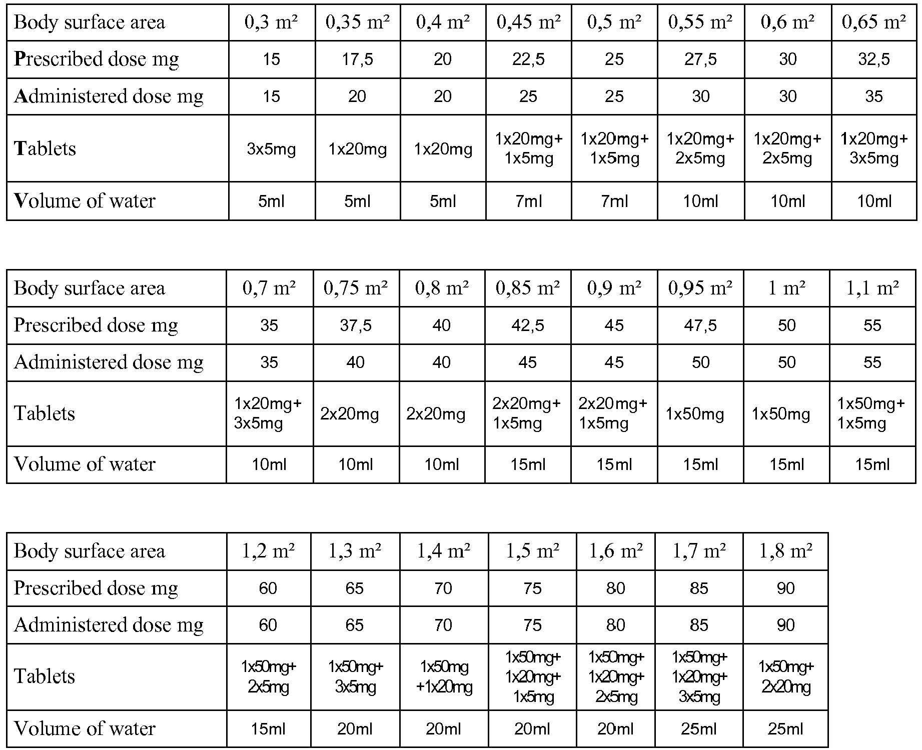

Depending on the volume of water used for dispersion of the tablet, the obtained solutions can present a cloudy aspect. This cloudy aspect may be observed for small volumes and is not related to cyclophosphamide as it dissolves completely, but comes from the presence of particles of insoluble excipients that can be present in the tablet depending on the particular formulation.

The tablets of the invention are stable, and can be kept several months, in particular at room temperature, or at 2 to 8°C (typically, in a fridge) depending on their composition.

In particular, tablets comprising cellulose or cellulose derivatives should be preferably stored in the fridge.

The tablets of the invention should be packaged in containers that reduce contact thereof with humidity, as cyclophosphamide is sensitive to the presence of water. Suitable packaging is for example: aluminium/aluminium blisters, glass bottles, polyethylene bottles with desiccant cap.

The tablets of the invention are preferably prepared by a dry blending of its ingredients, followed by a direct tabletting (or direct compression). Contact with water should be avoided during the manufacture of the tablets.

A preferred process for manufacturing the tablets of the invention comprises the following successive steps:
(1) thoroughly mixing the cyclophosphamide with said solubilising agent(s);
(2) adding the disintegrant, if any, and thoroughly mixing;
(3) adding the lubricant, if any, and thoroughly mixing;
(4) tabletting the mixture,
(5) packaging the tablets.

The tabletting is performed using classical tabletting machines used in the pharmaceutical industry.

### EXAMPLES :

The following dispersible tablets containing respectively 5 mg, 20 mg and 50 mg of cyclophosphamide, were prepared:

| Ingredients | Centesimal composition w/w | Dosage strength 5 mg | Dosage strength 20 mg | Dosage strength 50 mg |
|---|---|---|---|---|
| Cyclophosphamide | 6.25% | 5.00 mg | 20.00 mg | 50.00 mg |
| Mannitol | 66.55% | 53.24 mg | 212.96 mg | 532.40 mg |
| Microcrystalline Cellulose | 18.75% | 15.00 mg | 60.00 mg | 150.00 mg |
| Saccharin sodium | 2.00% | 1.60 mg | 6.40 mg | 16.00 mg |
| Croscarmellose sodium | 6.25% | 5.00 mg | 20.00 mg | 50.00 mg |
| Magnesium stearate | 0.20% | 0.16 mg | 0.64 mg | 1.60 mg |
| Total | 100% | 80.00 mg | 320.00 mg | 800.00 mg |

Several trials were performed, by dispersing the tablets above in water at room temperature. The time of disintegration of the tablets in water as well as the percentage of dissolution of cyclophosphamide (CPM) was determined.

The trials and results thereof are summarized in the table hereunder:

| Conditions | Trial 1 | Trial 2 | Trial 3 | Trial 4 | Trial 5 |
|---|---|---|---|---|---|
| Tablets | 1 tablet of 5 mg | 1 tablet of 50 mg | 2 tablets of 20 mg + 2 tablets of 5 mg | 2 tablets of 20 mg | 1 tablet of 20 mg |
| Water volume | 5 ml | 50 ml | 50 ml | 10 ml | 10ml |
| Dispersion Time | <1 min | <1 min | <1 min | <1 min | <1 min |
| Percentage of CPM dissolved | 92.4% | 91.7% | 96.1% | 100 % | 95.8% |

Whatever their dosage strength (5 mg, 20 mg and 50 mg), the tablets disclosed hereabove disintegrated in water at room temperature in less than 1 minute leading to a solution of cyclophosphamide. All the individual tablets as well as the tested tablets/doses combinations show more than 90% release of cyclophosphamide.

Trial N°4 corresponds to the expected in use conditions of the highest daily dose for administration (100mg/m²) to babies (6 months old) having body surface 0.4m². Trial N° 5 is representative of a routine daily dose administration in babies of 50 mg/m² during treatment. According to the clinical protocols, cyclophosphamide is generally to be administered on a routine basis at 50 mg/m² alone or 25 mg/m² when associated with vinorelbine. Therefore, based on the results of trial N°4 corresponding to 100 mg/m², it is possible to perform routine administration at 50 mg/m² in small babies with a very small volume of water (5 ml) or using 2.5 ml for a dose of 25mg/m².

In all cases, the dispersion and release of cyclophosphamide using the tablets of the invention appeared to be extremely fast leading to a cyclophosphamide solution. In addition, the obtained cyclophosphamide solutions allowed administering the needed doses using very small volumes of administration, which is particularly interesting for young babies.

The tablets of the invention also appeared to be fully and quickly dispersible in saliva, leading to a solution of cyclophosphamide in saliva. They can therefore be used as orodispersible tablets, which can typically be administered depending on the dosage strength to patients above 6 years old.

## Claims

1. Soluble, dispersible or orodispersible tablet, comprising:
(a) cyclophosphamide, a hydrate form thereof, a pharmaceutically acceptable salt thereof, or any mixture thereof, in an amount ranging from 1 to 15 % by weight on a cyclophosphamide active weight basis, and
(b) from 10 to 99 % by weight of a solubilising agent chosen from disaccharides, sugar alcohols that are solid at ambient temperature, and mixtures thereof.

2. The tablet of claim 1, wherein said disaccharide is lactose.

3. The tablet of claim 1, wherein said sugar alcohols contain at least 4 carbon atoms, more preferably at least 5 carbon atoms.

4. The tablet of the preceding claim, wherein said sugar alcohols are chosen from mannitol, sorbitol, xylitol, isomalt, maltitol, and mixtures thereof.

5. The tablet of any preceding claim, wherein the solubilising agent contains at least one sugar alcohol that is solid at ambient temperature, preferably mannitol.

6. The tablet of any preceding claim, wherein the amount of solubilising agent ranges from 10 to 98% by weight, preferably from 65 to 95 % by weight, more preferably from 70 to 90 % by weight.

7. The tablet of any preceding claim, wherein the cyclophosphamide is employed under the form of the monohydrate compound.

8. The tablet of any preceding claim, wherein the amount of cyclophosphamide, hydrate form(s) thereof, pharmaceutically acceptable salt(s) thereof, or mixture thereof, ranges from 2 to 10% by weight, preferably from 4 to 8 % by weight, on a cyclophosphamide active weight basis.

9. The tablet of the preceding claim, wherein the amount of cyclophosphamide, hydrate form(s) thereof, pharmaceutically acceptable salt(s) thereof, or mixture thereof is 6.25% by weight, on a cyclophosphamide active weight basis.

10. The tablet of any preceding claim, further comprising one or more lubricants.

11. The tablet of the preceding claim, wherein the lubricant is chosen from stearic acid as well as magnesium and calcium salts thereof, hydrogenated vegetable oil, mineral oil, polyethyleneglycol, sodium lauryl sulfate, glyceryl palmitostearate, glyceryl behenate, sodium benzoate, sodium stearyl fumarate, talc, silicon dioxide, and mixtures thereof, and is preferably magnesium stearate.

12. The tablet of anyone of claims 10 and 11, wherein the lubricant(s) is (are) present in an amount ranging from 0.1 to 5% by weight, preferably from 0.5 to 3 % by weight.

13. The tablet of any preceding claim, further comprising one or more disintegrants.

14. The tablet of the preceding claim, wherein the disintegrant is chosen from celluloses and modified celluloses, starches and modified starches, cross-linked polyvinylpyrrolidones, and mixtures thereof, and preferably from cross-linked carboxymethyl celluloses, carboxymethyl starches and mixtures thereof.

15. The tablet of anyone of claims 13 and 14, wherein the disintegrant(s) is (are) present in an amount ranging from 1 to 20% by weight, preferably from 3 to 15 % by weight.

16. The tablet of anyone of the preceding claims, **characterized in that** it does not contain microcrystalline cellulose.

17. The tablet of any preceding claim, containing from 5 to 100 mg of cyclophosphamide, on a cyclophosphamide active weight basis.

18. The tablet of any preceding claim, for its use in treating cancer or autoimmune disorders, in particular in children.

19. The tablet of the preceding claim, wherein it is administered orally after dispersing it in an aqueous solution or beverage, such as water, or after dispersing it in saliva.

20. Use of a solubilising agent chosen from disaccharides, sugar alcohols that are solid at ambient temperature, and mixtures thereof, for improving the dissolution of cyclophosphamide in aqueous solutions or beverages, such as water and saliva.

21. Use according to the preceding claim, wherein the solubilising agent is mannitol.
